# EUROPEAN PATENT APPLICATION

(11) **EP 2 741 118 A1**
(43) Date of publication of application: **11.06.2014**
(21) Application number: 13195610.4
(22) Date of filing: 04.12.2013
(51) Int. Cl.: G02B 23/16, G02B 23/24, G03B 11/04, G02C 7/16, A61F 9/04

(54) **Eye shielding apparatus**

(30) Priority: 05.12.2012 US 201261733659 P
(71) Applicant: Holding, Pace, Charlotte, North Carolina 28220 (US)
(72) Inventor: Holding, Pace, Charlotte, North Carolina 28220 (US)
(74) Representative: Hargreaves, Timothy Edward

(57) **Abstract**

In one aspect, eye shielding apparatus are described herein. In some embodiments, an eye shielding apparatus comprises an eye shield, a strap, and an arm connecting the eye shield to the strap, wherein the strap is operable to couple the eye shielding apparatus to an optical instrument. In some embodiments, the apparatus further comprises a secondary strap attached to the eye shield, wherein the secondary strap is operable to secure the eye shield to the side of the optical instrument.

## Description

### CROSS REFERENCE TO RELATED APPLICATION

This application claims priority pursuant to 35 U.S.C. § 119(e) to U.S. Provisional Patent Application Ser. No. 61/733,659, filed on December 5, 2012, which is hereby incorporated by reference in its entirety.

### FIELD

The present invention relates to eye shielding apparatus and optical instruments comprising eye shielding apparatus.

### BACKGROUND

Optical instruments having only one eyepiece, such as microscopes, telescopes, and monoculars, generally require users to partially or completely close the eye that is not used with the eyepiece. However, continual or repetitive squinting of one eye can lead to fatigue and/or significantly reduce the enjoyment or productivity of the viewing activity. Therefore, there exists a need for improved optical instruments and other apparatus that can reduce fatigue and provide superior enjoyment and/or productivity of various viewing activities.

### SUMMARY

In one aspect, eye shielding apparatus are described herein which, in some embodiments, may provide one or more advantages over other apparatus. For example, in some embodiments, an eye shielding apparatus described herein permits a user of a single-eyepiece optical instrument to block the vision of one eye without the need to squint or close the unused eye. Therefore, in some embodiments, an eye blocking apparatus described herein allows an individual to keep both eyes open while using a single-eyepiece optical instrument such as a microscope, telescope, or monocular, without sacrificing viewing quality. In addition, in some embodiments, an eye shielding apparatus described herein can have a structure that permits one or more components of the apparatus to be easily positioned for use or secured in a safe and convenient manner when not in use. Moreover, in some embodiments, an eye shielding apparatus described herein can be used with a variety of optical instruments having different sizes and/or shapes. Further, an eye shielding apparatus described herein, in some embodiments, can be adjustable and/or removable, permitting the apparatus to be reversibly attached to a variety of different optical instruments at different times in a modular way.

In some embodiments, an eye shielding apparatus described herein comprises an eye shield, a strap, and an arm connecting the eye shield to the strap, wherein the strap is operable to couple the eye shielding apparatus to an optical instrument. In some embodiments, the strap has a first end and a second end, the first end comprising a first fastener and the second end comprising a second fastener. When the first and second fasteners are coupled to one another, in some embodiments, the strap can form a continuous band or loop, including an adjustable loop.

Further, in some embodiments, the arm of an eye shielding apparatus described herein comprises a first segment and a second segment connected by a joint. The joint, in some embodiments, permits relative movement of the first and second segments in one, two, or three dimensions. In addition, in some embodiments, the arm is connected to the strap with a second joint and to the eye shield with a third joint. The second and third joints, in some embodiments, permit relative movement of the arm, strap, and eye shield in one or two dimensions. Thus, in some embodiments, the three joints of the eye shielding apparatus can together be used to position the eye shield in a desired position for use or storage of an optical instrument.

In another aspect, optical instruments are described herein which, in some embodiments, may provide one or more advantages over prior optical instruments. In some embodiments, for instance, an optical instrument described herein comprises only a single eyepiece but can nevertheless be used by a user while having both eyes open and/or without squinting. In some embodiments, an optical instrument described herein comprises an eyepiece and an eye shielding apparatus coupled to the optical instrument. The eye shielding apparatus comprises an eye shield, a strap, and an arm connecting the eye shield to the strap, wherein the strap couples the eye shielding apparatus to the optical instrument. In some embodiments, the eye shield of the eye shielding apparatus is disposed in a binocular position relative to the eyepiece.

In other embodiments, an optical instrument comprises an eyepiece and an eye shielding apparatus coupled to the optical instrument, wherein the eye shielding apparatus comprises an eye shield and an arm connecting the eye shield to the optical instrument directly, without the use of a strap. In some embodiments, the eye shielding apparatus is built-in to the optical instrument and/or is permanently coupled to the optical instrument.

These and other embodiments are described in greater detail in the detailed description which follows.

### BRIEF DESCRIPTION OF THE FIGURES

Figure 1 illustrates a perspective view of an eye shielding apparatus coupled to an optical instrument according to one embodiment described herein.
Figure 2A illustrates a perspective view of the eye shielding apparatus of Figure 1 during a folding and securing process.
Figure 2B illustrates a perspective view of the eye shielding apparatus of Figure 1 during a folding and securing process.
Figure 2C illustrates a perspective view of the eye shielding apparatus of Figure 1 during a folding and securing process.
Figure 2D illustrates a perspective view of the eye shielding apparatus of Figure 1 during a folding and securing process.
] Figure 3 illustrates a frontal view of an eye shielding apparatus coupled to an optical instrument according to one embodiment described herein.

### DETAILED DESCRIPTION

Embodiments described herein can be understood more readily by reference to the following detailed description and drawings. Elements, apparatus, and methods described herein, however, are not limited to the specific embodiments presented in the detailed description and drawings. It should be recognized that these embodiments are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" should generally be considered to include the end points 5 and 10.

### I. Eye Shielding Apparatus

In one aspect, eye shielding apparatus are described herein. In some embodiments, an eye shielding apparatus comprises an eye shield, a strap, and an arm connecting the eye shield to the strap, wherein the strap is operable to couple the eye shielding apparatus to an optical instrument. In some embodiments, the strap has a first end and a second end, wherein the first end comprises a first fastener and the second end comprises a second fastener. Any fasteners not inconsistent with the objectives of the present invention may be used. In some embodiments, for instance, a fastener comprises a hook and loop fastener. Typical hook and loop fasteners, in some embodiments, comprise VELCRO® or unbreakable loop backings (UBL). In other embodiments, a fastener comprises a button or snap. Moreover, the first and second fasteners, in some embodiments, can be coupled to one another. Alternatively, in other embodiments, the first and second fasteners can each be coupled to a portion of the optical instrument. The first and second fasteners of a strap described herein, in some embodiments, can thus be used to couple an eye shielding apparatus to an optical instrument. In some embodiments, a strap forms a continuous band when the first and second fasteners are coupled to one another. The continuous band, in some embodiments, forms a loop, and the loop can have an adjustable length or diameter. In some embodiments adjusting the length or diameter of a loop is accomplished by altering the point of attachment of one or more fasteners. Further, adjusting the length or diameter of a loop formed by the strap of an apparatus described herein, in some embodiments, permits the apparatus to be securely coupled or attached to an optical instrument, including a tubular optical instrument or a tubular portion of an optical instrument.

In other embodiments, a strap of an eye shielding apparatus described herein does not comprise a first end and a second end but instead comprises a continuous band forming a loop, such as a continuous elastic band forming an elastic or stretchable loop. In some embodiments comprising a strap comprising a continuous band forming a loop, the eye shielding apparatus can be coupled to an optical instrument by disposing the loop around a portion of the optical instrument, such as a tubular portion of the optical instrument.

In addition, in some embodiments, a strap of an eye shielding apparatus described herein comprises a gripping surface on at least one side of the strap. In some embodiments, the gripping surface is proximate or adjacent to a surface of an optical instrument. A gripping surface, for reference purposes herein, comprises a surface that includes one or more features that are operable to grip the surface of an optical instrument, such as by creating increased friction between the gripping surface and the surface of the optical element. Thus, a gripping surface can prevent or minimize slippage of the eye shielding apparatus, so that the relative positions of the eye shielding apparatus and the optical instrument remain unchanged or substantially unchanged during normal use of the optical instrument. In some embodiments, a gripping surface comprises one or more bumps, protrusions, or ridges. In some embodiments, a gripping surface is formed from a relatively high friction material such as rubber. Moreover, the strap of an eye shielding apparatus described herein can have any length, width, and thickness not inconsistent with the objectives of the present invention. In some embodiments, one or more dimensions of a strap are selected to optimize the ability of the strap to grip the optical element. In some embodiments, the width of a strap is between about 0.5 cm and about 10 cm, the length of a strap is between about 1 cm and about 20 cm, and the thickness of a strap is between about 0.1 cm and about 1 cm.

Further, eye shielding apparatus described herein comprise an arm connecting the strap to an eye shield. The arm can be connected to the strap and the eye shield in any manner not inconsistent with the objectives of the present invention. For example, in some embodiments, the arm is connected to the strap using a staple, screw, adhesive, snap, button, or other coupling means. In some embodiments, the arm is connected to the strap and the eye shield using a joint. Any joint not inconsistent with the objectives of the present invention may be used. For example, in some embodiments, a joint comprises a Hooke type universal joint, a ball and socket joint, or a heim joint. In addition, the arm of an eye shielding apparatus described herein, in some embodiments, comprises a plurality of segments connected by one or more joints, including one or more ball and socket joints. Alternatively, in other embodiments, an arm comprises only one segment such as one rigid segment.

Further, the joints of an eye shielding apparatus described herein, in some embodiments, permit relative movement of various components of the apparatus in a desired manner in one or more dimensions. In some embodiments, for instance, a joint between two arm segments permits relative movement of the segments in two or three dimensions. In some embodiments, a joint between an arm and a strap or between an arm and an eye shield permits relative movement of the components in one dimension. In some embodiments, a joint between an arm and a strap or between an arm and an eye shield permits relative movement in two dimensions or in three dimensions. Therefore, in some embodiments, the joints of an eye shielding apparatus described herein can be used to position the eye shield as needed or desired by a user for use of an optical instrument to which the apparatus is coupled, including for viewing objects through the optical instrument with one eye without the need to squint or close the other eye. Moreover, in some embodiments, one or more joints of the apparatus can be used to dispose the eye shield and/or other components of the apparatus in a compact or folded configuration, such as along the side of an optical instrument, when not in use. Thus, in some embodiments, an eye shielding apparatus described herein can be coupled to an optical instrument without substantially increasing the space needed to store the optical instrument.

In addition, in some embodiments, an eye shielding apparatus described herein further comprises a secondary strap attached to the eye shield. The secondary strap, in some embodiments, is operable to secure the eye shield of the apparatus to the side of the optical instrument. In this manner, additional compactness and/or security for transport or storage can be obtained. A secondary strap can have any structure and can be coupled to the eye shield in any manner not inconsistent with the objectives of the present invention. In some embodiments, for instance, a secondary strap has one or more features of a strap described hereinabove and/or is coupled to the eye shield in a manner described hereinabove.

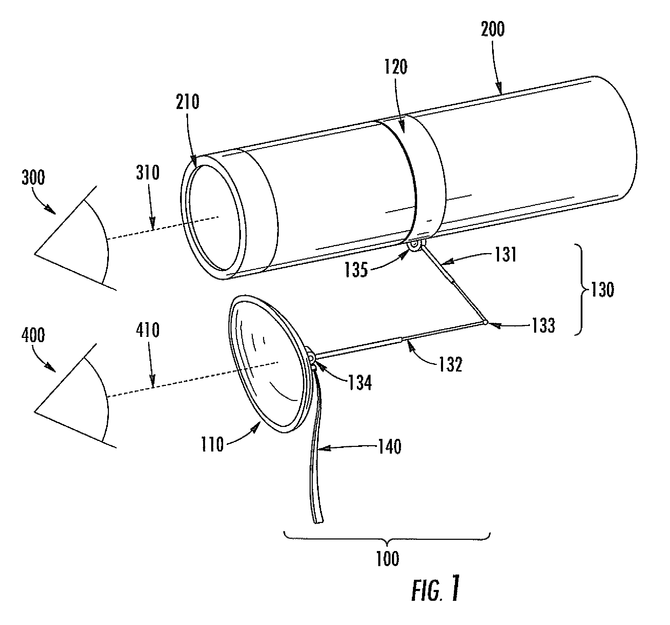

Turning now to the figures, Figure 1 illustrates a perspective view of an eye shielding apparatus coupled to an optical instrument according to one embodiment described herein. As illustrated in Figure 1, an eye shielding apparatus (100) is coupled to an optical instrument (200). The optical instrument (200) comprises a single eyepiece (210) at one end for viewing purposes. The eye shielding apparatus (100) comprises an eye shield (110), a strap (120), and an arm (130) connecting the eye shield (110) to the strap (120). The strap (120) couples the eye shielding apparatus (100) to the optical instrument (200). In the embodiment of Figure 1, the eye shielding apparatus (100) further comprises a secondary strap (140) attached to the eye shield (110). As illustrated in Figure 1, the secondary strap (140) is not being used to secure the eye shield (110) to the optical instrument (200). Instead, the secondary strap (140) is depicted in a loose or unused state. However, as described further herein, the secondary strap (140) can be used to secure one or more components of the eye shielding apparatus (100). In the embodiment of Figure 1, the optical instrument (200) is a monocular. However, other optical instruments can also be used. For example, in some embodiments, the optical instrument is a telescope. In other embodiments, the optical instrument is a microscope.

As illustrated in Figure 1, the arm (130) of the eye shielding apparatus (100) comprises a first segment (131) and a second segment (132). The first and second segments (131, 132) are connected by a joint (133). However, other structures are also possible. For example, in some embodiments, the arm (130) comprises only a single segment. Similarly, as illustrated in Figure 1, the first and second segments (131, 132) of the arm (130) have different lengths, and the first segment (131) is shorter than the second segment (132). However, in other embodiments, the first and second segments (131, 132) have the same or substantially the same length. Moreover, in some embodiments, the relative lengths of the segments (131, 132) can be varied. For example, in some embodiments, one or more segments can be telescoping segments. In the embodiment of Figure 1, the second segment (132) is telescoping. Therefore, the length of the telescoping second segment (132) can be altered as desired by a user, such as for positioning the eye shield in a preferred location along the telescoping dimension, as described further hereinbelow. In some embodiments, the extended state of the telescoping second segment (132) can be up to about twice the length of the contracted state of the telescoping second segment (132). In other embodiments, the extended state is more than twice the length of the contracted state, such as two to four times or three to five times the length of the contracted state. Further, in some embodiments, the length of the first segment (131) is no greater than about half or no greater than about one-third of the length of the second segment (132) in an extended state. In some embodiments, the ratio of the length of the first segment (131) to the length of the second segment (132) in an extended state is between about 1:1 and about 1:5. Moreover, in some embodiments, the length of the first segment (131) is the same or substantially the same (e.g., within about 10 percent) as the length of the second segment (132) in the contracted state.

Further, a segment of an arm (130) can be formed from any material not inconsistent with the objectives of the present invention. For example, in some embodiments, a segment such as the first and/or second segment (131, 132) is formed from a rigid material. In other embodiments, a segment is formed from a flexible material. In some embodiments, a segment is formed from plastic or metal. In some embodiments, a metal segment comprises a rigid metal rod or tube or a flexible metal wire. Further, metal wire, in some embodiments, comprises coated metal wire such as plastic or rubber coated metal wire.

In the embodiment of Figure 1, the arm (130) is coupled to the eye shield (110) with a second joint (134) and to the strap (120) with a third joint (135). As described further herein, the various joints (133, 134, 135) can be used to position the eye shield (110) and/or one or more other components of the eye shielding apparatus (100) as desired by a user for use of the optical instrument (200) or for storage or transportation of the eye shielding apparatus (100) or optical instrument (200). For example, as illustrated in Figure 1, the eye shield (110) has been positioned in a binocular relationship to the eyepiece (210) of the optical instrument (200). For reference purposes herein, components having a binocular relationship or position are arranged so that one component (such as the eyepiece (210)) occupies the center of the field of vision of one eye of a normal human user and the other component (such as the eye shield (110)) occupies the center of the field of vision of the other eye of the user. For example, as illustrated in Figure 1, the eyepiece (210) of the optical instrument (200) occupies the center of the field of vision of a first eye (300) of a user and the eye shield (110) of the eye shielding apparatus (100) occupies the center of the field of vision of a second eye (400) of the user, as indicated by the optical paths (310, 410). The first optical path (310) extends between the first eye (300) and the eyepiece (210). The second optical path (410) extends between the second eye (400) and the eye shield (410). Thus, in some embodiments, an eye shielding apparatus (100) described herein can be used to block the vision of an unused eye (400) of a user during operation of an optical instrument (200), thereby obviating the need for the user to squint or close the unused eye (400) and thus relieving eye strain or muscle fatigue. Further, in some embodiments, the components of an eye shielding apparatus can be positioned without the use of one or more specific joints. For example, in some embodiments wherein a segment of an arm is formed from a flexible material, such as a flexible metal wire, the position of the eye shield of the apparatus can be altered by bending the flexible arm segment.

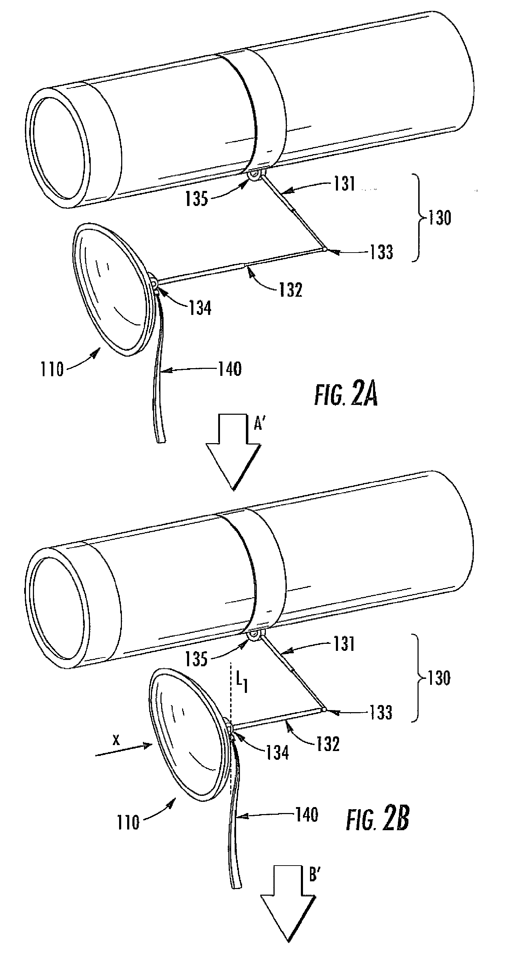

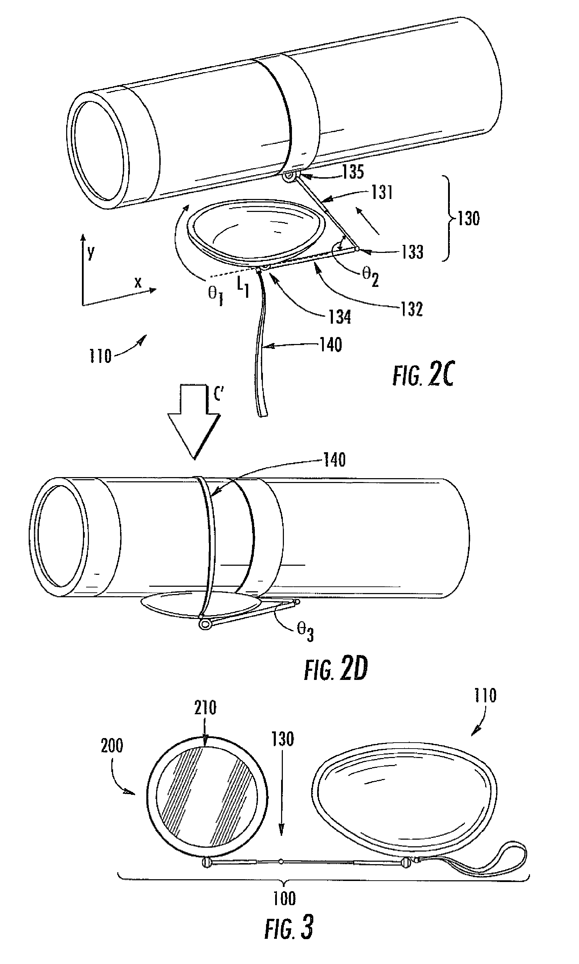

Figure 2 illustrates a perspective view of the eye shielding apparatus (100) of Figure 1 during a folding and securing process. For clarity purposes, most of the reference numerals in Figure 1 have been omitted in Figure 2. However, it is understood that like numerals correspond to like features. As illustrated in Figure 2, the eye shielding apparatus is coupled to an optical instrument in a position (A) for normal use of the optical instrument by a user. After use, the eye shielding apparatus of Figure 2 can be folded and secured to the optical instrument for storage, transportation, or for another purpose. In the first folding and securing step (A'), the length of the telescoping second segment (132) of the arm (130) is reduced by contracting the second segment (132) along the telescoping direction (x) to provide a contracted position (B). In the next step (B'), the eye shield (110) is rotated through an angle (θ₁) in the xy-plane using the second joint (134) to provide a folded position (C), wherein the angle (θ₁) is defined by the beginning and ending positions of a line (L₁) parallel to the plane of the eye shield (110) at the second joint (134). In some embodiments, the angle (θ₁) ranges between about 1 degree and about 180 degrees or between about 1 degree and about 90 degrees. In the final step (C') illustrated in Figure 2, the folded eye shield (110) is moved into contact with the side of the optical instrument by bringing together the first and second segments (131, 132) of the arm (130) through an angle (θ₂) in the xy-plane using joint (133) and rotating the first segment (131) through an angle (θ₃) using second joint (135). The angle (θ₂) is defined by the beginning and ending positions of the first arm segment (131) relative to the second arm segment (132), and the angle (θ₃) is defined by the beginning and ending positions of the first arm segment (131) relative to the adjacent surface of the optical instrument. In some embodiments, the angle (θ₂) and/or the angle (θ₃) ranges between about 1 degree and about 180 degrees or between about 1 degree and about 90 degrees. In addition, in the final step (C'), the secondary strap (140) is disposed around the optical instrument to secure the eye shielding apparatus in a folded and secured position (D). It should be noted that the non-limiting folding and securing process illustrated in Figure 2 is an exemplary process only. As understood by one of ordinary skill in the art, the various components of an eye shielding apparatus described herein can be used to carry out other folding and/or securing processes, including processes utilizing angles and directions different than those illustrated in Figure 2.

Figure 3 illustrates a frontal view of an eye shielding apparatus coupled to an optical instrument according to one embodiment described herein, as seen by a user. In the embodiment of Figure 3, an eye shielding apparatus (100) described herein is coupled to an optical instrument (200) comprising a single eyepiece (210). The eye shielding apparatus (100) comprises an eye shield (110), a strap (not shown), and an arm (130) connecting the strap to the eye shield (110). Further, as illustrated in Figure 3, the eye shield (110) is disposed in a binocular relationship to the eyepiece (210).

In the embodiment of Figure 3, the eye shield (110) has an oval shape or a substantially oval shape. An oval or substantially oval shape, in some embodiments, comprises a shape approximating the shape of a human eye socket or the shape of a common lens for eyeglasses or sunglasses. However, the eye shield of an eye shielding apparatus described herein may also have a different shape. Any shape not inconsistent with the objectives of the present invention may be used. For example, in some embodiments, an eye shield has a round or circular shape. In other embodiments, an eye shield has a rectangular or square shape. Moreover, an eye shield of an eye shielding apparatus described herein can be formed of any material not inconsistent with the objectives of the present invention. In some embodiments, for instance, an eye shield is formed from a soft material, including a material that will not scratch or otherwise damage the surface of an optical instrument. In some embodiments, an eye shield is formed from an elastic material and/or a durable material. In some embodiments, an eye shield is formed from a textile, metal, plastic, rubber, or foam material. A rubber material can include a natural or a synthetic rubber such as neoprene. Further, in some embodiments, an eye shield has a composite construction and is formed from a plurality or combination of materials, including a plurality of materials described herein.

### II. Optical Instruments

In another aspect, optical instruments are described herein. In some embodiments, an optical instrument comprises an eyepiece and an eye shielding apparatus coupled to the optical instrument. The eye shielding apparatus comprises an eye shield, a strap, and an arm connecting the eye shield to the strap, wherein the strap couples the eye shielding apparatus to the optical instrument. The eye shielding apparatus of the optical instrument can comprise any eye shielding apparatus described in Section I hereinabove. Further, in some embodiments, the eye shield of the eye shielding apparatus is disposed in a binocular position relative to the eyepiece of the optical instrument. An eye shield disposed in a binocular position relative to the eyepiece, in some embodiments, fills or substantially fills the field of vision of the unused eye of the user of the optical instrument. The unused eye, for reference purposes herein, comprises the eye that is not centered on the eyepiece of the optical instrument. Further, an optical instrument, in some embodiments, comprises only one eyepiece. For example, in some embodiments, an optical instrument comprises a monocular, a telescope, or a microscope.

In addition, in some embodiments, the eye shielding apparatus of an optical instrument described herein can be permanently attached to the optical instrument rather than being a modular and removable component. Such a built-in eye shielding apparatus can have any of the features of an eye shielding apparatus described in Section I hereinabove not inconsistent with the objectives of the present invention. In some embodiments, an optical instrument comprises an eyepiece and an eye shielding apparatus coupled to the optical instrument, wherein the eye shielding apparatus comprises an eye shield and an arm connecting the eye shield to the optical instrument. The eye shielding apparatus can be coupled to the optical instrument in any manner not inconsistent with the objectives of the present invention. In some embodiments, for example, the arm of the eye shielding apparatus is directly affixed or attached to the optical instrument using a joint or other attachment means described herein. In some embodiments, the arm of the eye shielding apparatus is formed as a unitary part of the optical instrument. Moreover, in some embodiments, the eye shield of an eye shielding apparatus described herein is disposed in a binocular position relative to the eyepiece of the optical instrument, which, in some embodiments, comprises only one eyepiece. For instance, in some embodiments, an optical instrument comprises a monocular, telescope or microscope.

Various embodiments of the invention have been described in fulfillment of the various objectives of the invention. It should be recognized that these embodiments are merely illustrative of the principles of the present invention. Numerous modifications and adaptations thereof will be readily apparent to those skilled in the art without departing from the spirit and scope of the invention.

That which is claimed is:

## Claims

1. An eye shielding apparatus comprising:
an eye shield;
a strap; and
an arm connecting the eye shield to the strap,
wherein the strap is operable to couple the eye shielding apparatus to an optical instrument.

2. The apparatus of claim 1 further comprising a secondary strap attached to the eye shield, wherein the secondary strap is operable to secure the eye shield to the side of the optical instrument.

3. The apparatus of claim 1 or 2, wherein the eye shield is formed from a textile, metal, plastic, rubber, or foam material or a combination thereof.

4. The apparatus of any preceding claim, wherein the strap has a first end and a second end, the first end comprising a first fastener and the second end comprising a second fastener.

5. The apparatus of claim 4, wherein the first and second fasteners are operable to couple to one another.

6. The apparatus of claim 5, wherein the first and second fasteners are operable to couple the eye shielding apparatus to the optical instrument.

7. The apparatus of claim 5 or 6, wherein the strap forms a continuous band when the first and second fasteners are coupled to one another.

8. The apparatus of claim 7, wherein the continuous band forms a loop, and optionally:-
the length or diameter of the loop is adjustable.

9. The apparatus of any preceding claim, wherein the strap comprises a continuous band forming a loop, and
optionally:-
the loop is elastic.

10. The apparatus of any preceding claim, wherein:-
the strap comprises a gripping surface; and/or
the arm comprises a first segment and a second segment connected by a joint.

11. The apparatus of claim 10, wherein:-
the first segment or the second segment is telescoping; and/or
the joint permits relative movement of the first and second segments in at least two dimensions.

12. An optical instrument comprising:
an eyepiece; and
an eye shielding apparatus coupled to the optical instrument, the eye shielding apparatus comprising:
an eye shield;
a strap; and
an arm connecting the eye shield to the strap,
wherein the strap couples the eye shielding apparatus to the optical instrument.

13. The optical instrument of claim 12, wherein:-
the eye shield of the eye shielding apparatus can be disposed in a binocular position relative to the eyepiece; and/or
the optical instrument comprises a monocular or a telescope.

14. An optical instrument comprising:
an eyepiece; and
an eye shielding apparatus coupled to the optical instrument, the eye shielding apparatus comprising:
an eye shield; and
an arm connecting the eye shield to the optical instrument.

15. The optical instrument of claim 14, wherein the optical instrument comprises a monocular or a telescope.
